# EUROPEAN PATENT SPECIFICATION

(11) **EP 0 514 411 B1**
(45) Date of publication and mention of the grant of the patent: **09.05.2001**
(21) Application number: 91903249.0
(22) Date of filing: 04.02.1991
(51) Int. Cl.: C12N 15/30, A61K 39/015, A61K 39/395, C12P 21/08, C12N 15/62, C12N 15/86, C12N 15/10

(54) **POLYPEPTIDES AND DNA ENCODING SAME, ASSOCIATED WITH HUMAN MALARIA PARASITES**
POLYPEPTIDE UND DAFÜR KODIERENDE DNS, DIE MIT HUMANEN MALARIAPARASITEN ASSOZIIERT SIND
POLYPEPTIQUES ET ADN CODANT POUR CES DERNIERS, ASSOCIES AUX PARASITES DE LA MALARIA CHEZ L' TRE HUMAIN

(30) Priority: 05.02.1990 GB 9002512
(43) Date of publication of application: 25.11.1992
(73) Proprietor: IMPERIAL COLLEGE INNOVATIONS LIMITED, London SW7 2AZ (GB)
(72) Inventor: ROBSON, Kathryn Jane Hallowes, Long Hanborough, Oxford OX7 2BP (GB)
(74) Representative: Sheard, Andrew Gregory
(86) International application number: GB9100162
(87) International publication number: WO9111516

(56) References cited:
- EP-A- 0 283 829
- EP-A- 0 329 257
- WO-A-84/02917
- WO-A-90/01496
- Nature, vol. 355, 1 September 1988, (London, GB), K.J.H. Robson et al.: "A highly conserved amino-acid sequence in thrombospondin, properdin and in proteins from sporozoites and blood stages of a human malaria parasite", pages 79-82

## Description

This invention relates to polypeptides, and to DNA encoding same, associated with human malaria parasites. It also relates to methods of preparing the polypeptides, to antibodies thereto and compositions for use against malaria.

Plasmodium falciparum malaria is one of the most common infectious diseases in the world today, threatening up to 40% of the world's population. It is a disease of the Third World. There are between 150 and 300 million cases of this disease annually, over 1% of cases are fatal, babies and young children being the most vulnerable. With the advent of insecticides and new parasiticidal drugs developed after World War II it was felt that the disease could be eradicated. The early attempts proved very successful but with time the parasite has developed resistance to drugs such as chloroquine and the mosquito vector (Anopheles) has developed resistance to DDT. As a consequence of this it is necessary to develop new approaches to try to combat the disease. As immunity to the disease develops with increasing age, in endemic areas, a vaccine, together with new anti-malarials and insecticides need to be developed if the disease is to be eradicated.

Current research programmes, throughout the world, are involved in defining what antigens might form part of a useful vaccine. The complex life-cycle of the parasite means that a simple vaccine based on one antigen may not be adequate and that an effective vaccine will probably require antigens from different development stages.

The human malaria parasite, Plasmodium falciparum, has a complex life-cycle, during which different antigens are produced at particular developmental stages. The major antigen on the sporozoite surface is the circumsporozoite or CS protein, which probably determines the specificity of the interaction between the parasite and liver cells. CS protein contains two conserved amino acid sequences, known as regions I and II, which are separated by a repeating amino acid motif.

The cloning of the gene for this protein has permitted the development of various vaccines. To date vaccine trials using parts of the CS protein have proved disappointing. Immunity to sporozoites does not necessarily prevent the erythrocytic phase of the life-cycle which is associated with clinical disease. Only one sporozoite needs to evade the immune system for clinical disease to occur. Currently CS protein is the only well-characterised protein known to be involved in host-cell recognition. The merozoite is the developmental stage capable of re-infecting fresh red cells. Antibodies which prevent gametocyte differentiation within the mosquito are useful in breaking the transmission cycle as well. Another complexity is the antigenic variation displayed by the parasite. A vaccine against the asexual erythrocytic parasite, need only be partially effective to reduce the severity of the disease. A vaccine against the asexual blood stages of P. falciparum has been developed by Patarroyo et al (Nature Vol.332, 1988, p158) based on the use of synthetic peptides, but this has not proved to be totally effective.

Robson et al (Nature. Vol 335 pp 79-82, 1988) have reported that polypeptides sharing certain sequence motifs with CS protein are produced during the erythrocytic stage of the parasite life-cycle. Such polypeptides and DNA encoding therefor also form the subject matter of co-pending published patent application PCT/GB 89/00895 (International Publication No. WO90/01496). One example is illustrated in formula I of the said patent application and in figure 2 on page 80 of the said Nature publication and is referred to therein and hereinafter by the abbreviation "TRAP" (thrombospondin-related anonymous protein). The numbering system used herein for amino acid and nucleotide residues is the same as that of the said figure 2.

Comparisons of the nucleotide sequence in DNA's from several different isolates of P. falciparum have now been made. Considerable variation between isolates has been found, as would be expected for malaria, but one region of the DNA molecule has been found to be completely conserved in the isolates examined. It is likely, therefore, that the polypeptide sequence coded for by this region is significant for the biological properties of the polypeptide as a whole.

Accordingly, the present invention provides a polypeptide consisting of the amino acid residue sequence 182-276 inclusive represented in Formula I or of such a sequence incorporating one or more conservative amino acid changes.

Further the present invention provides a polypeptide consisting of the amino acid residue sequence 221-276 inclusive represented in Formula I or of such a sequence incorporating one or more conservative amino acid changes.

Formula I represents a region of the "TRAP" protein which, for the purposes of the above definition, may be derived from any source (and is not confined to protein from isolate T9/96 - Thailand) and which includes the polypeptide sequences 182-276 (overlined) and 221-276 of the invention. Sequence 182-276 is hereinafter referred to as the conserved sequence or region and sequence 221-276 as the inner conserved sequence or region respectively.

Although the present invention is based on the surprising discovery of the high degree of conservation within the above region of the "TRAP" molecule, it will be understood by those skilled in the art that the biological activity of this region may be tolerant of some changes in composition. Such changes, however, should be of a subtle or conservative character.

Biological activity of interest may include, for example, immunogenic activity, involvement in parasite recognition of red cells, red cell attachment or merozoite invasion. Activity may also include involvement in binding to sulphated glycoconjugates.

The single letter symbols for amino acid residues (upper row) in Formula I represent the following naturally occurring L-amino acids: (A) alanine, (C) cysteine, (D) aspartic acid, (E) glutamic acid, (F)phenylalanine, (G) glycine, (H) histidine, (I) isoleucine, (K) lysine, (L) leucine, (M) methionine, (N) asparagine, (P) proline, (Q) glutamine, (R) arginine, (S) serine, (T) threonine, (V) valine, (W) tryptophan, (Y) tyrosine.

Derivatives of the polypeptides of the invention are, for example, where functional groups, such as amino, hydroxyl, mercapto or carboxyl groups, are derivatised, e.g. glycosylated, acylated, amidated or esterified, respectively. In glycosylated derivatives an oligosaccharide is usually linked to asparagine, serine, threonine and/or lysine. Acylated derivatives are especially acylated by a naturally occurring organic or inorganic acid, e.g. acetic acid, phosphoric acid or sulphuric acid, which usually takes place at the N-terminal amino group, or at hydroxy groups, especially of tyrosine or serine, respectively. Esters are those of naturally occurring alcohols, e.g. methanol or ethanol.

Further derivatives are salts, especially pharmaceutically acceptable salts, for example metal salts, such as alkali metal and alkaline earth metal salts, e.g. sodium, potassium, magnesium, calcium or zinc salts, or ammonium salts formed with ammonia or a suitable organic amine, such as a lower alkylamine, e.g. triethylamine, hydroxy-lower alkylamine, e.g. 2-hydroxyethylamine, and the like.

The present invention also provides DNA sequences coding for the polypeptides of the invention.

DNA according to the present invention may be recovered from malaria parasite DNA and genomic libraries by methods known in the art and it will be understood that, since the sequence is known, direct amplification is possible, by the polymerase chain reaction, for example. (Saiki et al, Science 1985 Vol. 230 pp1350-1354 and PCR Technology. Ed. Erlich, Stockton Press, New York, USA, 1989.)

The polypeptides of the invention may be prepared by methods known in the art, for example, by chemical synthesis or by expression of the appropriate DNA sequences in a host/vector expression system.

Recombinant vectors comprising the appropriate DNA, together with other functional sequences, such as promoter and marker genes, may be made by methods known in the art.

Suitable vectors include recombinant plasmids comprising a DNA sequence of the present invention cloned, for example, into M13mp8 (Amersham), pUC13 (Pharmacia) or pAc YM1, (Inst. of Virology, Mansfield Road, Oxford, England).

Recombinant viral vectors may be obtained by incorporating the appropriate DNA sequence into viral DNA by methods known in the art. (See, for example, DNA Cloning, Volume II, D.M. Glover, published 1985, IRL Press, Oxford, England, and Molecular Cloning, A Laboratory Manual, 2nd Edition, Sambrook, Fritsch and Maniatis, Cold Spring Harbour, USA.) One suitable method, according to the present invention, involves the combination of a plasmid with a virus using a co-transfection process in a suitable host cell.

Using a suitable plasmid, for example, together with the Autographa californica Nucleopolyhedrosis Virus (AcNPV) in Spodoptera frugiperda cells, recombinant virus containing DNA sequence of the present invention may be reproducibly isolated.

A plasmid pKKJl7 comprising the "TRAP" sequence has been deposited at the National Collections of Industrial and Marine Bacteria Limited (NCIMB), 23 St. Machar Drive, Aberdeen, AB2 1RY, United Kingdom, under Accession No. NCIMB 40164 and a virus vKKJl7 expressing the sequence has been deposited at the European Collection of Animal Cell Cultures (ECACC), Public Health Service Laboratory, Centre for Applied Microbiology and Research, Porton Down, Salisbury, Wiltshire, SP4 OJG, United Kingdom, under Accession No. 89071402. Both deposits were made on 14th July, 1989, in connection with Patent Application PCT/GB89/00895 (WO 90/01496).

According to a further aspect of the present invention we provide polyclonal or monoclonal antibodies to the polypeptides of the invention. The antibodies may be made by techniques known in the art (see for example: Antibodies, A Laboratory Manual, E. Harlow and D. Lane, Cold Spring Harbour 1988) and may be used, for example, to purify the polypeptides of the invention, as a passive vaccine for the treatment of patients with malaria and as diagnostic reagents.

The polypeptides of the invention are likely to be useful in the preparation of vaccines and the like against malaria. For this purpose they may be incorporated as active ingredients in suitable carriers, adjuvants, etc. possibly in combination with other immunologically active materials to provide protection against different stages of the malaria parasite.

They may also be useful as antagonists for inhibition of sequestration or of invasion of cells by the parasite.

The invention will now be described in more detail with reference to the accompanying formula and tables in which:

| | |
|---|---|
| Formula I | represents a region of the "TRAP" protein from malarial isolate T9/96 (Thailand) including a polypeptide sequence according to the invention (overlined) and DNA coding therefor; |
| Table I | lists the changes in deduced amino acid residues across a range of malarial parasite variants, relative to a consensus sequence, derived from isolate FCR3 (Rockerfeller); |
| Table II | sets out the origins of the parasite variants of Table I; |
| and Table III | lists changes in DNA corresponding to the changes in the amino acid residue listed in Table I. |

Reference should also be made to the numbering system for the amino acid and nucleotide residues for "TRAP" used in Figure 2 of the Nature paper referred to above (Vol. 335, 1st September 1988, page 80).

Referring now to Formula I, a short region of "TRAP" from T9/96 and associated DNA is represented, including the polypeptide sequence of the present invention with short additional sequences at each end. It has been found from DNA sequence analysis of ten laboratory isolates and three wild isolates that the deduced amino acid residue sequence from 182 to 276 inclusive is completely conserved, suggesting that it is significant for biological activity and may form the basis for approaches to the treatment or prevention of malaria. It is believed that the inner conserved sequence 221-276 in particular is conserved in malarial isolates.

Apart from this long conserved sequence it has been found that individual changes in nucleotide and amino acid residues occur with the relatively high frequency which is typical of other malarial genes. It will be noted, however, that there are further, shorter conserved sequences which may also be significant for biological activity, for example between acid residues 362 and 397, 422 and 451 and between 491 and 525.

Table I lists the amino acid residue changes using the "consensus" sequence of laboratory isolate, FCR3, as a reference sequence. It should be noted that the strain described in Formula I and Figure 2 of the cited Nature paper is T9/96. Except where indicated in Table 1,FCR3 is the same as T9/96.

Table III lists changes in nucleotide residues of DNA, corresponding to the amino acid residue changes set out in Table 1. It is a summary of the amino acid substitutions in the form of nucleotide changes and where these occur within particular codons. There is only one silent nucleotide change; this is in K1 and is a T - C transition in the third position of the codon for Cys²⁷³ and does not, as a consequence, alter the amino acid.

No alterations occur in the 95 amino acid residues in the conserved region, suggesting it is important for biological activity. It will be understood, however, by those skilled in the art that even so, there may be some toleration of minor variations of a subtle or conservative character. Such possibilities may include, for example, the substitution of T for S at position 251, of K for R at position 266 or of D for E at position 276.

### Experimental Details

### 1. Isolation and sequencing of DNA from different isolates

The polymerase chain reaction was used for the direct isolation of characterised genes from genomic DNA.

Two oligonucleotide primers were designed containing BamH 1 sites as well as the 5' and 3' ends of the "TRAP" genes. The sequences of these two primers were: A    GGATCCAAAATAATGAATCATCTTGGG B GGATCCGTATTATATTTAATTCCACTCG the underlined sequences correspond to the end of the coding sequence. Primer A represents the coding strand and in primer B this is the complementary strand. The DNA amplified was a genomic malaria DNA. The reaction was set up as described by Saiki et al (Science 1985, 230, 1350-1354) utilising Tag 1 polymerase (1 mM oligonucleotide primers A and B, 10mM deoxynucleoside triphosphates and 100 ng of genomic malaria DNA). The cycle times and temperatures were 1' at 93°C, 1' at 37°C, 5' at 72°C, the numbers of cycles was 35. After amplification the complete reaction mix was extracted with phenol/CHCl₃, followed by gel filtration to remove the unused deoxynucleoside triphosphates. The ends of the DNA product were repaired using the Klenow fragment of deoxyribonuclease 1 and fresh deoxynucleoside triphosphates. The reaction was again terminated by phenol/CHCl₃ extraction and gel filtration using Sephadex G50/80. The 5' ends of the DNA were phosphorylated using T4 polynucleotide kinase and adenosine triphosphate. The reaction was terminated by phenol/CHCl₃ extraction and the DNA recovered by propan-2-ol precipitation. This material was used as the substrate of a ligation reaction using T4 DNA ligase and phosphatased Sma 1 cut M13mp8 (Amersham). Constructs containing the desired insert were obtained and their authenticity checked by DNA sequencing.

### 2. Isolation and amplification of DNA from conserved fragment

The following exemplary scheme for the isolation and sequencing of DNA coding for polypeptides of the invention was carried out.

Two different primers for the polymerase chain reaction were constructed as follows:

GGATCCATGGGTGTTAAAATAGCTGTTTTTGGTATTGGAC (coding strand)

GGATCCTTATTCACTTGTACATCCTTCGTGTAAG (complementary strand)

The polymerase chain reaction conditions were adjusted to give a 2' extension time at 72°C as the amplified product is considerably shorter than 1.7 kb. The template for amplification is a subclone containing this sequence such as a RV9 (a subclone of "Trap" in M13).

The reaction was set up as described by Saiki et al (Science 1985, 230, 1350-1354) utilising Taq 1 polymerase [(1 mM) oligonucleotide primers A and B, 10 mM deoxynucleoside triphosphates and 1 ng of RV9. The cycle times and temperatures were 1' at 93°C, 1' at 37°C, 2' at 72°C, the number of cycles was 15-20. After amplification the complete reaction mix was extracted with phenol/CHCl₃, followed by gel filtration to remove the unused deoxynucleoside triphosphates. The ends of the DNA product were repaired using the Klenow fragment of deoxyribonuclease 1 and fresh deoxynucleoside triphosphates. The reaction was terminated by phenol/CHCl₃ extraction and gel filtration using Sephadex G50/80. The 5' ends of the DNA were phosphorylated using T4 polynucleotide kinase and adenosine triposphate. The reaction was terminated by phenol/CHCl₃ extraction and the DNA recovered by propan-2-ol precipitation. This material was used as the substrate of a ligation reaction using T4 DNA ligase and phosphatased Sma 1 cut pUCl3 (Pharmacia) and phosphatased Sma 1 cut M13mp8 (Amersham). Constructs containing the desired insert were obtained and their authenticity was checked by DNA sequencing.

A minimum of two M13 clones containing the "TRAP" sequence were examined by dideoxy sequencing using a series of primers which enabled rapid analysis of the amplified and cloned DNA.

### 3. Expression of polypeptide

The constructs produced in 2 above can be used to produce polypeptides of the invention by the following proposed exemplary scheme.

The fragment generated by digestion of the construct described with the restriction enzyme BamH 1 can be cloned into the BamH 1 site of pAcYMl.

The fragment is suitably released from pUC13 by BamH 1 digestion demonstrating that the necessary restriction sites would permit transfer of the transplacement plasmid pAcYMl (obtainable from the Institute of Virology, Oxford, England). A suitable construct could be digested with BamH 1 and Hae 111, the reaction terminated by phenol/CHCl₃ extraction, followed by ethanol precipitation. This digest can be carried out
to prevent gel purification of the desired 300bp BamH 1 fragment prior to religation with the Bam HI site of pAcYMl. A similar ligation and transformation can be carried out with the insert and the new vector pAcYMl. Again constructs containing the desired fragment can be obtained. The orientation of the insert containing the "TRAP" sequence relative to the polyhedrin promoter is suitably checked by restriction mapping and sequencing.

Using pAcYMl containing the desired DNA sequence a transfection of Spodoptera frugiperda cells may be performed as outlined below. 25ug of the appropriate plasmid together with 1 ug of caesium choloride purified Autographa californica Nucleopolyhedrosis virus (AcNPV) DNA are prepared in Hepes buffered saline pH 7.5 containing 10 mM glucose and 125 mM CaCl₂. A calcium/DNA complex is allowed to form over a 45 minute period prior to addition to freshly plated Spodoptera frugiperda cells (1.5 x 10⁶ cells) and incubation for 1 hr at room temperature. The DNA precipitate is removed, fresh medium (TC100) added and the cells incubated at 20°C until a cytopathic effect is observed (3 days post transfection). The viruses produced by these cells should be both wild type AcNPV and recombinant AcNPV containing the "TRAP" gene. Plaque purification following titration permits the isolation of pure recombinant virus. This is facilitated by the fact that wild type AcNPV has intact polyhedrin gene and so produces occluded plaques whereas recombinants do not. The differences between the two types of plaque can be visualised using light microscopy.

The new construct suitably produces a soluble protein which is releasable from infected cells by lysis with detergents or sonication. Further purification using monoclonal antibodies to this region should be possible. Alternatively, affinity chromatography on heparin agarose should permit purification of the conserved fragment.

### 4. Synthetic process for polypeptide

As an alternative to the processes proposed above, the polypeptides of the invention may be made by chemical synthetic methods from component amino acids.

Examples of such methods are described in "Peptide Synthesis" by Atherton, E., Clive, D.J.L., and Sheppard, R.C., J.C.S. Perkin 529-537 (1981).

A more specific example is described by Schneider, J. and Kent, S.B.H. (1988) Cell 54 363-8 who describe enzymatic activity of a synthetic 99 residue protein corresponding to the putative HIV-1 protease and by Wlodawer et al (1989) Science 245 616-621, who elucidate certain features of the crystal structure of the protease using X-ray crystallographic techniques.

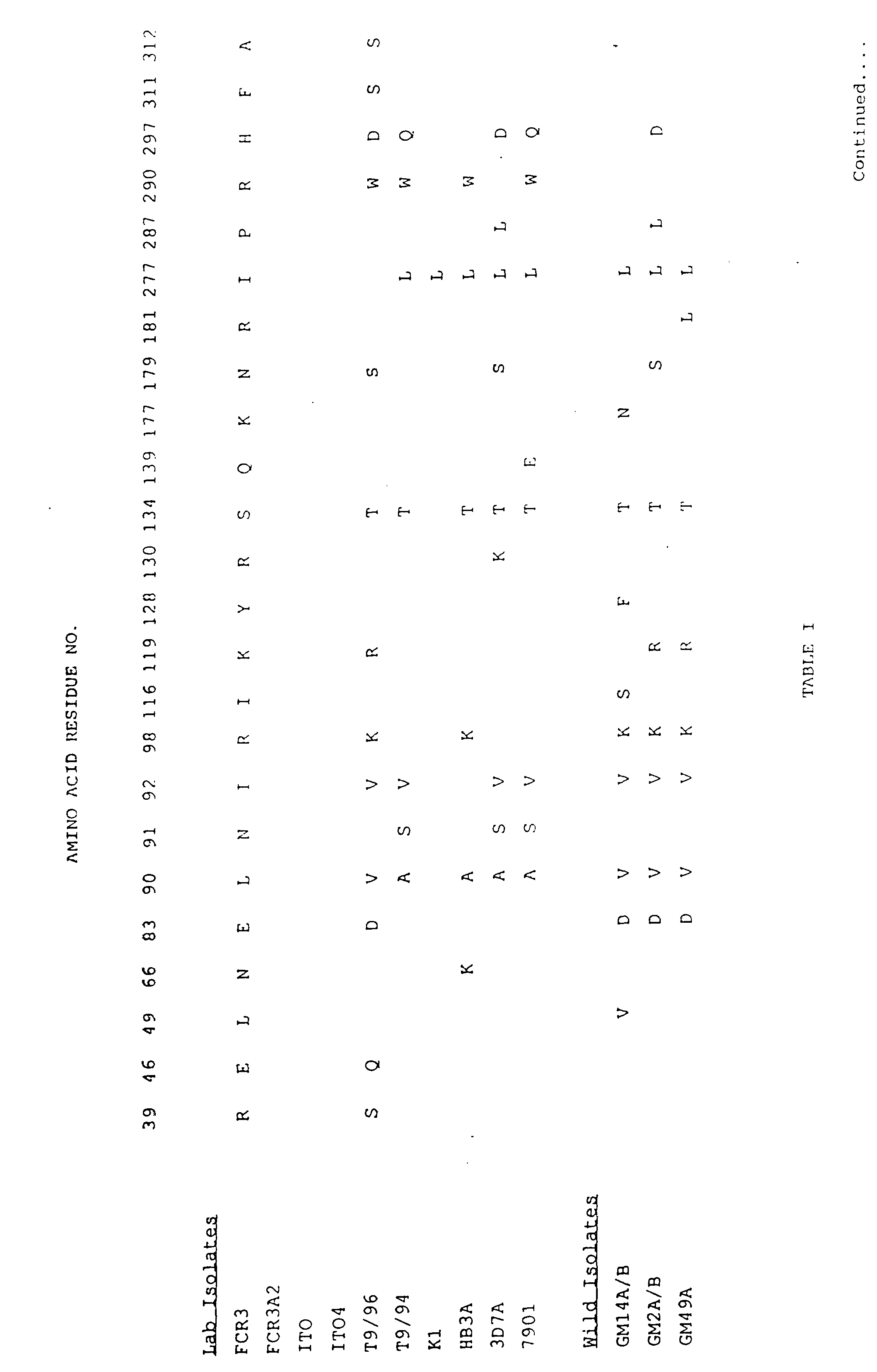

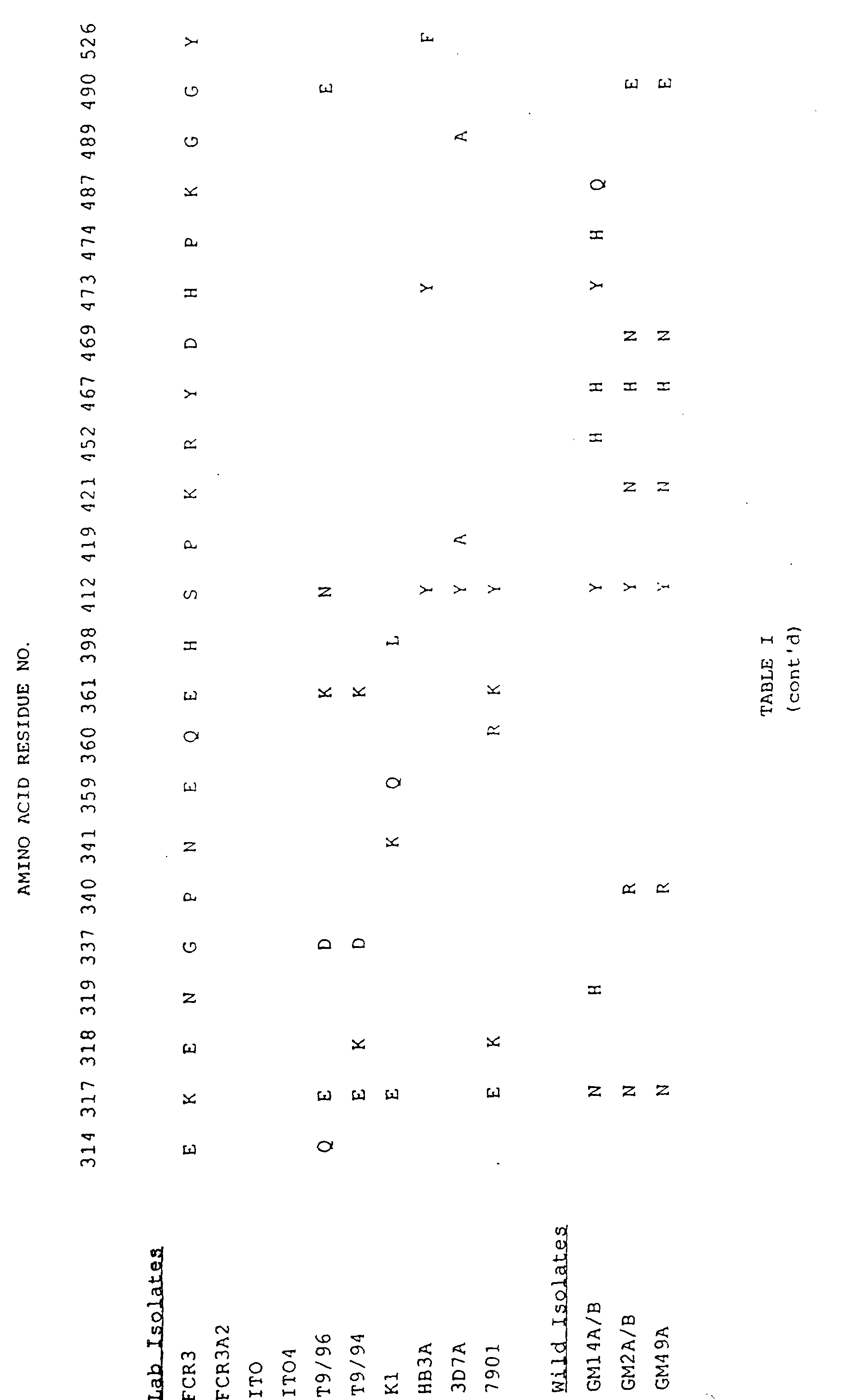

171 181
Q D S L K E S R K L S D R G V K I A V
CAA GAT TCA TTA AAA GAA TCA AGA AAA TTA AGT GAT CGT GGT GTT AAA ATA GCT GTT
191 201
F G I G Q G I N V A F N R F L V G C H
TTT GGT ATT GGA CAA GGT ATT AAT GTA GCT TTC AAC AGA TTT CTT GTA GGT TGT CAT
211 221
P S D G K C N L Y A D S A W E N V K N
CCA TCA GAT GGT AAA TGT AAC TTG TAT GCT GAT TCT GCA TGG GAA AAT GTA AAA AAT
231 241
V I G P F M K A V C V E V E K T A S C
GTT ATC GGA CCC TTT ATG AAG GCT GTT TGT GTT GAA GTA GAA AAA ACA GCA AGT TGT
251 261
G V W D E W S P C S V T C G K G T R S
GGT GTT TGG GAC GAA TGG TCT CCA TGT AGT GTA ACT TGT GGT AAA GGT ACC AGG TCA
271 281
R K R E I L H E G C T S E I Q E Q C E
AGA AAA AGA GAA ATC TTA CAC GAA GGA TGT ACA AGT GAA ATA CAA GAA CAA TGT GAA

FORMULA I

**Table II**

| Parasite Isolates | Origin |
|---|---|
| T9/96 | Thailand |
| T9/94 | Thailand |
| K1 | Thailand |
| HB3A | Honduras |
| 3D7A | The Netherlands NF54 (Africa) |
| ITO | Brazil |
| ITO4 | Selected form of ITO |
| FCR3 | Gambia |
| FCR3A2 | clone FCR3 |
| 7901 (Palo Alto) | Uganda |
| T9/96, T9/94, HB3A, 3D7A are all obtainable from David Walliker, WHO Registry of Standard Strains of Malaria Parasite, Dept. of Genetics, University of Edinburgh, United Kingdom.. The other isolates can be obtained from The Rockefeller University in New York. The DNA samples with GM designations were from children with malaria in the Gambia. | |

**TABLE III**

| Amino Acid residue number | Position of change in codon | Nucleotide change | Type of change |
|---|---|---|---|
| 39 | 1 | C -> A | Transversion |
| 46 | 1 | G -> C | Transversion |
| 49 | 1 | C -> G | Transversion |
| 66 | 3 | C -> A | Transversion |
| 83 | 3 | A -> T | Transversion |
| 90A (GCT) | 1 and 2 | C -> G, T -> C | Transition, Transversion |
| 90V (GTT) | 1 | C -> G | Transversion |
| 91 | 2 | A -> G | Transition |
| 92 | 1 | A -> G | Transition |
| 98 | 2 | G -> A | Transition |
| 116 | 2 | T -> G | Transversion |
| 119 | 2 | A -> G | Transition |
| 128 | 2 | A -> T | Transversion |
| 130 | 2 | G -> A | Transition |
| 134 | 2 . | G -> C | Transversion |
| 139 | 1 | C -> G | Transversion |
| 177 | 3 | A -> T | Transversion |
| 179 | 2 | A -> G | Transition |
| 181 | 2 | G -> T | Transversion |
| 277 | 1 | A -> T | Transversion |
| 287 | 2 | C -> T | Transition |
| 290 | 1 | T -> C | Transition |
| 297D (GAT) | 1 | C -> G | Transversion |
| Q (CAA) | 3 | T -> A | Transversion |
| 311 | 2 | T -> C | Transition |
| 312 | 1 | G -> T | Transversion |
| 314 | 1 | G -> C | Transversion |
| 317 E (GAA) N (AAC) | 1 3 | A -> G A -> C | Transition Transversion |
| 318 | 1 | G -> A | Transition |
| 319 | 1 | A -> C | Transversion |
| 337 | 2 | G -> A | Transition |
| 340 | 2 | C -> G | Transversion |
| 341 | 3 | C -> A | Transversion |
| 359 | 1 | G -> C | Transversion |
| 360 | 2 | A -> G | Transition |
| 361 | 1 | G -> A | Transition |
| 398 | 2 | A -> T | Transversion, Transversion |
| 412 N (AAT) Y (TAT) | 1 and 2 1 | T -> A, C -> A C -> A | Transversion Transversion |
| 419 | 1 | C -> G | Transversion |
| 421 | 3 | A -> C | Transversion |
| 452 | 2 | G -> A | Transition |
| 467 | 1 | T -> C | Transition |
| 469 | 1 | G -> A | Transition |
| 473 | 1 | C -> T | Transition |
| 474 | 2 | C -> A | Transversion |
| 487 | 1 | A -> C | Transversion |
| 489 | 2 | G -> C | Transversion |
| 490 | 2 | G -> A | Transition |
| 526 | 2 | A -> T | Transversion |

## Claims

1. A polypeptide consisting of the amino acid residue sequence 182-276 inclusive represented in Formula I or of such a sequence incorporating one or more conservative amino acid changes.

2. A polypeptide consisting of the amino acid residue sequence 221-276 inclusive represented in Formula I or of such a sequence incorporating one or more conservative amino acid changes.

3. A polypeptide according to claim 1 or claim 2 which consists of the amino acid residue sequence 182-276 inclusive or 221-276 inclusive, represented in Formula I.

4. A DNA sequence coding for a polypeptide as claimed in any of claims 1 to 3.

5. A recombinant vector comprising a DNA sequence as claimed in claim 4.

6. A vector as claimed in claim 5 which is a recombinant plasmid.

7. A vector as claimed in claim 6 in which the plasmid is an M13mp8, pUC13 or pAc YM1 plasmid.

8. A vector as claimed in claim 5 which is a recombinant virus or variant or mutant thereof.

9. A vector as claimed in claim 8 in which the virus is *Autographa californica* Nucleopolyhedrosis Virus.

10. A host/vector system capable of expressing a DNA sequence as claimed in claim 4 and comprising a vector as claimed in any one of claims 5-9.

11. A host/vector system as claimed in claim 10 in which the host is selected from the species *Spodoptera frugiperda.*

12. A method for the preparation of a polypeptide as claimed in claim 1, claim 2 or claim 3 comprising the expression of a DNA sequence as claimed in claim 4 in a host/vector expression system.

13. A method as claimed in claim 12 which comprises the use of a vector as claimed in any one of claims 5-9.

14. A method as claimed in claim 13 which comprises the use of a host/vector system as claimed in claim 10 or 11.

15. A method of preparing a vector as claimed in claim 8 or claim 9 comprising the steps of:
a) preparing a plasmid containing a DNA sequence as claimed in claim 4;
b) co-transfecting a host cell with DNA from the said sequence and from a virus;
c) recovering recombinant virus containing the said DNA sequence.

16. A method as claimed in claim 15 in which the plasmid is a vector as claimed in any one of claims 5 to 7.

17. A method as claimed in claim 16 in which the virus is the *Autographa californica* Nucleopolyhedrosis Virus.

18. A method as claimed in any one of claims 15-17 in which the host cell is of the species *Spodoptera frugiperda.*

19. A pharmaceutical composition comprising as an active constituent a polypeptide as claimed in claim 1, claim 2 or claim 3 in a pharmacologically acceptable vehicle.

20. The use of a polypeptide as claimed in claim 1, 2 or 3 or a DNA sequence as claimed in claim 4, in the preparation of a prophylactic or therapeutic agent against malaria.

21. A polyclonal or monoclonal antibody capable of binding to a polypeptide according to any of claims 1 to 3.

22. The use of an antibody according to claim 21 to purify a polypeptide according to any of claims 1 to 3.

23. An antibody according to claim 21 for use in medicine.

24. The use of an antibody according to claim 21 in the manufacture of a passive vaccine for treating malaria.

25. A passive vaccine against malaria comprising an antibody according to claim 21.

26. The use of an antibody according to claim 21 as a diagnostic reagent.

## Patentansprüche

1. Polypeptid, das aus der Aminosäurerestsequenz 182 bis einschließlich 276, die in Formel I dargestellt ist, besteht, oder aus einer derartigen Sequenz, die eine oder mehrere konservative Aminosäureaustausche beinhaltet.

2. Polypeptid, das aus der Aminosäurerestsequenz 221 bis einschließlich 276, die in Formel I dargestellt ist, besteht, oder aus einer derartigen Sequenz, die eine oder mehrere konservative Aminosäureaustausche beinhaltet.

3. Polypeptid nach Anspruch 1 oder Anspruch 2, das aus der Aminosäurerestsequenz 182 bis einschließlich 276 oder 221 bis einschließlich 276 besteht, die in Formel I dargestellt ist.

4. DNA-Sequenz, die für ein Polypeptid nach einem der Ansprüche 1 bis 3 kodiert.

5. Rekombinanter Vektor, der eine DNA-Sequenz nach Anspruch 4 aufweist.

6. Vektor nach Anspruch 5, der ein rekombinantes Plasmid ist.

7. Vektor nach Anspruch 6, in dem das Plasmid ein M13mp8, pUC13 oder pAc YM1 Plasmid ist.

8. Vektor nach Anspruch 5, der ein rekombinantes Virus oder eine Variante oder Mutante davon ist.

9. Vektor nach Anspruch 8, in dem das Virus das Autographa californica Nucleopolyhedrose Virus ist.

10. Wirt/Vektorsystem, das zur Expression einer DNA-Sequenz nach Anspruch 4 imstande ist und einen Vektor nach einem der Ansprüche 5 - 9 aufweist.

11. Wirt/Vektorsystem nach Anspruch 10, in dem der Wirt aus der Spezies *Spodoptera frugiperda* ausgewählt ist.

12. Verfahren für die Herstellung eines Polypeptids nach Anspruch 1, Anspruch 2 oder Anspruch 3, das die Expression einer DNA-Sequenz nach Anspruch 4 in einem Wirt/Vektor-Expressionssystem umfaßt.

13. Verfahren nach Anspruch 12, das die Verwendung eines Vektors nach einem der Ansprüche 5 - 9 umfaßt.

14. Verfahren nach Anspruch 13, das die Verwendung eines Wirt/Vektorsystems nach Anspruch 10 oder 11 umfaßt.

15. Verfahren zur Herstellung eins Vektors nach Anspruch 8 oder Anspruch 9, das die Schritte umfaßt:
a) Herstellen eines Plasmids, das eine DNA-Sequenz nach Anspruch 4 enthält;
b) Co-Transfektieren einer Wirtszelle mit DNA von dieser Sequenz und von einem Virus;
c) Gewinnen des rekombinanten Virus, das die DNA-Sequenz enthält.

16. Verfahren nach Anspruch 15, in dem das Plasmid ein Vektor nach einem der Ansprüche 5 bis 7 ist.

17. Verfahren nach Anspruch 16, in dem das Virus das Autographa californica Nucleopolyhedrose Virus ist.

18. Verfahren nach einem der Ansprüche 15 - 17, in dem die Wirtszelle von der Spezies *Spodoptera frugiperda* ist.

19. Pharmazeutische Zusammensetzung, die als Wirkstoff ein Polypeptid nach Anspruch 1, Anspruch 2 oder Anspruch 3 in einem pharmakologisch annehmbaren Träger umfaßt.

20. Verwendung eines Polypeptids nach Anspruch 1, 2 oder 3 oder einer DNA-Sequenz nach Anspruch 4 in der Herstellung eines prophylaktischen oder therapeutischen Mittels gegen Malaria.

21. Polyklonaler oder monoklonaler Antikörper, der zur Bindung an ein Polypeptid nach einem der Ansprüche 1, 2 oder 3 imstande ist.

22. Verwendung eines Antikörpers nach Anspruch 21 zur Reinigung eines Polypeptids nach einem der Ansprüche 1 bis 3.

23. Antikörper nach Anspruch 21 zur Verwendung in der Medizin.

24. Verwendung eines Antikörpers nach Anspruch 21 zur Herstellung eines passiven Impfstoffes zur Behandlung von Malaria.

25. Passiver Impfstoff gegen Malaria, umfassend einen Antikörper nach Anspruch 21.

26. Verwendung eines Antikörpers nach Anspruch 21 als Diagnostikum.

## Revendications

1. Polypeptide constitué de la séquence de résidus d'acides aminés 182-276 inclus représentée dans la formule I ou ayant une séquence de ce type incorporant un ou plusieurs changements d'acides aminés conservateurs.

2. Polypeptide constitué de la séquence de résidus d'acides aminés 221-276 inclus représentée dans la formule I ou ayant une séquence de ce type incorporant un ou plusieurs changements d'acides aminés conservateurs.

3. Polypeptide selon la revendication 1 ou 2, constitué de la séquence de résidus d'acides aminés 182-276 inclus, ou 221-276 inclus, représentée dans la formule I.

4. Séquence d'ADN codant pour un polypeptide selon l'une quelconque des revendications 1 à 3.

5. Vecteur recombinant comprenant une séquence d'ADN selon la revendication 4.

6. Vecteur selon la revendication 5, qui est un plasmide recombinant.

7. Vecteur selon la revendication 6, dans lequel le plasmide est un plasmide M13mp8, pUC13 ou pAc YM1.

8. Vecteur selon la revendication 5, qui est un virus recombinant ou une variante ou un mutant de celui-ci.

9. Vecteur selon la revendication 8, dans lequel le virus est le virus de nucléopolyédrose *Autographa californica.*

10. Système hôte/vecteur capable d'exprimer une séquence d'ADN selon la revendication 4 et comprenant un vecteur selon l'une quelconque des revendications 5-9.

11. Système hôte/vecteur selon la revendication 10, dans lequel l'hôte est choisi dans l'espèce *Spodoptera frugiperda.*

12. Procédé de préparation d'un polypeptide selon la revendication 1, 2 ou 3, comprenant l'expression d'une séquence d'ADN selon la revendication 4 dans un système d'expression hôte/vecteur.

13. Procédé selon la revendication 12, qui comprend l'utilisation d'un vecteur selon l'une quelconque des revendications 5-9.

14. Procédé selon la revendication 13, qui comprend l'utilisation d'un système hôte/vecteur selon la revendication 10 ou 11.

15. Procédé de préparation d'un vecteur selon la revendication 8 ou 9, comprenant les étapes consistant :
a) à préparer un plasmide contenant une séquence d'ADN selon la revendication 4;
b) à co-transfecter une cellule hôte avec de l'ADN provenant de ladite séquence et d'un virus; et
c) à récupérer le virus recombinant contenant ladite séquence d'ADN.

16. Procédé selon la revendication 15, dans lequel le plasmide est un vecteur selon l'une quelconque des revendications 5 à 7.

17. Procédé selon la revendication 16, dans lequel le virus est le virus de nucléopolyédrose *Autographa californica.*

18. Procédé selon l'une quelconque des revendications 15-17 dans lequel la cellule hôte est de l'espèce *Spodoptera frugiperda*.

19. Composition pharmaceutique comprenant comme constituant actif un polypeptide selon la revendication 1, 2 ou 3 dans un véhicule pharmacologiquement acceptable.

20. Utilisation d'un polypeptide selon la revendication 1, 2 ou 3 ou d'une séquence d'ADN selon la revendication 4, dans la préparation d'un agent prophylactique ou thérapeutique contre la malaria.

21. Anticorps polyclonal ou monoclonal capable de se lier à un polypeptide selon l'une quelconque des revendications 1 à 3.

22. Utilisation d'un anticorps selon la revendication 21 pour purifier un polypeptide selon l'une quelconque des revendications 1 à 3.

23. Anticorps selon la revendication 21 pour un usage en médecine.

24. Utilisation d'un anticorps selon la revendication 21 dans la fabrication d'un vaccin passif pour le traitement de la malaria.

25. Vaccin passif contre la malaria comprenant un anticorps selon la revendication 21.

26. Utilisation d'un anticorps selon la revendication 2 comme réactif de diagnostic.
